# EUROPÄISCHE PATENTSCHRIFT

(11) **EP 0 485 393 B1**
(45) Veröffentlichungstag und Bekanntmachung des Hinweises auf die Patenterteilung: **05.06.1996**
(21) Anmeldenummer: 90909627.3
(22) Anmeldetag: 29.06.1990
(51) Int. Cl.: B01D 69/12, B01D 67/00, B01D 71/00, C07C 7/144, C10G 31/11

(54) **MEMBRAN ZUM AUSTRAG UNGESÄTTIGTER KOHLENWASSERSTOFFE SOWIE VERFAHREN ZU IHRER HERSTELLUNG**
MEMBRANE FOR EXTRACTING UNSATURATED HYDROCARBONS AND PROCESS FOR OBTAINING IT
MEMBRANE POUR L'EXTRACTION D'HYDROCARBURES NON SATURES ET PROCEDE DE FABRICATION

(30) Priorität: 20.07.1989 DE 3924102
(43) Veröffentlichungstag der Anmeldung: 20.05.1992
(73) Patentinhaber: GKSS-FORSCHUNGSZENTRUM GEESTHACHT GMBH, D-21502 Geesthacht (DE)
(72) Erfinder: PEINEMANN, Klaus-Victor, D-2057 Reinbek (DE); SHUKLA, Satish-Kumar, D-2058 Lauenburg (DE); BEHLING, Rolf-Dieter, D-2000 Hamburg 55 (DE)
(74) Vertreter: Niedmers, Ole, Dipl.-Phys.
(86) Internationale Anmeldenummer: DE9000490
(87) Internationale Veröffentlichungsnummer: WO9101177

(56) Entgegenhaltungen:
- EP-A- 0 144 054
- EP-A- 0 191 209
- GB-A- 2 169 300
- GB-A- 2 169 301
- US-A- 3 773 844

## Beschreibung

Die Erfindung betrifft eine Membran zum Austrag ungesättigter Kohlenwasserstoffe aus gasförmigen und flüssigen Stoffgemischen, insbesondere Kohlenwasserstoffgemischen, mit einer als Trenn- oder Sperrschicht dienenden ersten Schicht, die aus einer wasserhaltigen Lösung eines organischen Polymers und eines Metallsalzes erhältlich ist, sowie einer zweiten mikroporösen Schicht, deren Poren und/oder wenigstens Porenöffnungen von der ersten Schicht abgedeckt sind, sowie ein Verfahren zu ihrer Herstellung.

Eine Membran ähnlicher Art ist bekannt (GB-A-2 169 301). Bei dieser bekannten einschichtigen Membran werden zum Aufbau der Membran Polyalkohole oder Mischungen aus Polyalkoholen verwendet. Polyalkohole sind wasserlöslich, wobei dementsprechend auch Membranschichten, die aus Polyalkoholen gebildet werden bzw. diese enthalten, wasserlöslich sind. Die Folge ist, daß auf den bekannten Membranen Wasser während der Gastrennung kondensieren kann mit dem Ergebnis, daß die Membran sich auflöst. Die Folge ist, daß die bekannte Membran somit nicht stabil ist und somit für eine Anwendung, bei der Wasserlöslichkeit Voraussetzung ist, nicht geeignet ist.

Ungesättigte Kohlenwasserstoffe sind reaktive Verbindungen und werden in der chemischen Industrie zur Herstellung zahlreicher Verbindungen verwendet. In der Öl- und Petrochemie enthalten Produktströme aus den Crackverfahren in der Regel Olefine, deren Abtrennung beispielsweise für die Einstellung der Oktanzahl eines Vergaserkraftstoffs oder für die Weiterverarbeitung erforderlich ist. Es besteht daher ein erhebliches wirtschaftliches Interesse, ungesättigte Kohlenwasserstoffe aus Kohlenwasserstoffgemischen abzutrennen. Wenn die Kohlenwasserstoffmischungen flüssig anfallen oder leicht verflüssigt werden können, können normalerweise bekannte Destillationsverfahren zur Trennung eingesetzt werden. Oft ist jedoch die destillative Trennung schwierig, da die Siedepunkte der gesättigten und der ungesättigten Kohlenwasserstoffe vielfach nahe beieinander liegen. In einigen Fällen, wie beispielsweise n-Butan/ 1,3-Butadien, existieren auch azeotrope Gemische, die durch einfache Destillation gar nicht getrennt werden können. Oftmals werden die ungesättigten Kohlenwasserstoffe abgetrennt, indem man sie mit einem selektiven Lösemittel extrahiert, um dann in einem zweiten Schritt von dem Lösemittel durch Destillation getrennt zu werden. Diese Lösemittel können entweder organische Verbindungen, wie z.B. N-Methylpyrrolidon, Dimethylformamid oder Dimethylacetamid sein, es können aber auch wäßrige Lösungen bestimmter Übergangsmetalle eingesetzt werden.

Es ist bekannt, daß Ionen der Übergangsmetalle mit ungesättigten Kohlenwasserstoffen reversibel zu sogenannten π-Komplexen reagieren können. Besonders hervorzuheben sind Ag⁺- und Cu⁺-Ionen.

Bei bekannten Prozessen der vorbeschriebenen Art handelt es sich grundsätzlich um mehrstufige Verfahren, d.h. die ungesättigten Kohlenwasserstoffe werden bei einem bestimmten Druck und einer bestimmten Temperatur absorbiert und dann in einem zweiten Schritt bei vermindertem Druck oder erhöhter Temperatur wieder desorbiert. Nachteilig bei diesen Prozessen ist, daß sie sehr aufwendig sind und in der Regel große Mengen von sehr teuer bereitstellbaren Metallsalzen benötigt werden.

Es ist versucht worden, diese bekannten mehrstufigen Verfahren in einem kontinuierlichen Verfahren auszuführen, indem die entsprechenden Metallsalze in Membranen eingelagert wurden. Dabei wurden beispielsweise auf der Zulaufseite der Membran Bedingungen eingestellt, unter denen ein Metall-Olefin-Komplex gebildet wurde. Auf der Produktseite, d.h. auf der Permeatseite der Membran, wurden durch verminderten Druck Bedingungen geschaffen, unter denen der gebildete Komplex wieder zerfällt. Auf diese Weise konnte der Prozeß zwar kontinuierlich durchgeführt werden und es konnte auf der anderen Seite die benötigte Menge an Metallsalz erheblich vermindert werden, derartige Prozesse und die dabei verwendeten Membranen erforderten jedoch nachteiligerweise, daß die Membranen während des Betriebes "wassernaß" gehalten werden müssen. Wenn diese Membranen austrocknen, verlieren sie vollständig ihre Selektivität, wodurch grundsätzlich der technische Einsatz derartiger Membranen stark erschwert oder unmöglich gemacht wird. Hinzu kommt noch, daß der Zulaufstrom zur Membran wasserdampfgesättigt werden muß, und es darf kein Wasser auf der Membran auskondensieren. Ein weiterer wesentlicher Nachteil derartiger bekannter wassernasser Membranen ist der, daß diese keine hohe Druckdifferenz aufzunehmen vermögen, da die Gefahr des Ausblasens oder Ausdampfens des Wassers besteht.

Aus der US-A-4 780 114 ist eine Membran bekannt geworden, mit der die voraufgezeigten Nachteile vermindert werden sollten, indem kein freies Wasser in der Membran zugelassen wurde. Statt dessen wurden geschmolzene Salzhydrate in die Membran eingelagert, wobei das vorhandene Wasser hierin an die vorhandenen Ionen gebunden wurde. Im einzelnen wurde dort eine mikroporöse Membran mit geschmolzenem Calciumnitrattetrahydrat oder Silbernitrat gefüllt, wobei die erreichte Ethen/ Ethan-Selektivität mit 4,7 allerdings verhältnismäßig gering war.

In der DE-A-35 46 323 ist ebenfalls eine wasserfreie Membran zur Abtrennung von Kohlenwasserstoffen beschrieben worden, wobei Membranen aus Nafion (Produktname der Fa. Du Pont) verwendet wurden. Nafion bezeichnet perfluorierte Ionenaustauscher mit -SO₃-H⁺-Gruppen. Die Protonen der Nafionmembranen werde durch Ag⁺-Ionen ausgetauscht und die Membran wurden mit Glycerin behandelt. Auch mit dieser Membran wurden bei verhältnismäßig niedrigen Flüssen Ethen/Ethan-Selektivitäten von lediglich 8 bis 15 erzielt.

Es ist Aufgabe der vorliegenden Erfindung, eine wasserfreie Membran zu schaffen, mit der aus Flüssigkeiten und Gasen ungesättigte Kohlenwasserstoffe mit sehr hoher Selektivität ausgetragen werden können, sowie ein Verfahren zur Herstellung einer derartigen Membran zu schaffen, das einfach ausführbar ist, so daß derartige Membranen kostengünstig bereitstellbar sind.

Gelöst wird die Aufgabe gemäß der Erfindung dadurch, daß bei der Membran das organische Polymer aus einem Ionenaustauscher besteht und das Massenverhältnis Metallsalz zu organischem Polymer 0,1 bis 9 beträgt.

Der Vorteil der erfindungsgemäßen Membran liegt im wesentlichen darin, daß diese die Ausbildung einer sehr hohen Salzkonzentration im organischen Polymer der ersten Schicht gestattet, die im Vergleich zur oben genannten, aus der DE-A-35 46 323 bekannten Nafionmembran, bei der das Nafion eine Ionenaustauschkapazität von 0,91 mequiv/g aufweist und sich darin maximal 98 mg Silber in 1 g Nafion unterbringen lassen, beispielsweise die Unterbringung von 4,5 g Silbertetrafluoroborat pro g Nafion zuläßt. Die hohe Salzkonzentration ist somit erfindungsgemäß die Ursache für die extrem hohe Selektivität der erfindungsgemäßen Membran.

Obwohl die Membran im vorbeschriebenen Sinne schon in bezug auf die erwartete Selektivität ausreichend ist, ist es gemäß einer besonderen Ausgestaltung der Membran vorteilhaft, daß die erste Schicht aus einem Metallsalz und einem Polymer die zweite Schicht vollständig, d.h. nicht nur die Poren bzw. die Porenöffnungen der zweiten Schicht, abgedeckt. Bei dieser Ausgestaltung der Membran weist die erste Schicht vorteilhafterweise eine Dicke von 5 x 10⁻⁴-10⁻² mm auf.

Bei einer anderen vorteilhaften Ausgestaltung dient zur Bildung der ersten Schicht eine wäßrige Polymerlösung oder eine wäßrige Polymerdispersion, die das Metallsalz in gelöster Form enthalten, und zwar mit einer derart extrem feinen Verteilung der Metallsalze, daß selbst bei einer Vergrößerung von 3 x 10⁴ mit dem Rasterelektronenmikroskop keine Kristalle erkennbar sind.

Das Polymer wird vorzugsweise aus einer Polyolefindispersion gebildet, wobei das Polymer vorzugsweise Polyvinylidenchlorid enthält.

Bei einer noch anderen vorteilhaften Ausgestaltung der Membran ist die Polymerlösung eine Nafionlösung.

Als Metallionen können sowohl vorzugsweise die Ionen der Edelmetalle als auch der Nichtedelmetalle des Periodensystems der Elemente ausgewählt werden, wobei es sich insbesondere als vorteilhaft erwiesen hat, als Metallionen Silbermetallionen zu verwenden, mit denen beispielsweise eine Buten/Butan-Selektivität erreicht wurde, die bisher von keiner bekannten Membran auch nur annähernd erreicht wurde.

Grundsätzlich gilt, daß für das Metallsalz jedes geeignete Anion ausgewählt werden kann. Es hat sich jedoch als vorteilhaft herausgestellt, als Anion Bortetrafluorid (BF₄⁻) auszuwählen, obwohl grundsätzlich das Bortetrafluorid-Anion auch durch Nitrat, Perchlorat oder Trifluormethansulfat ersetzt werden kann.

Die zweite Schicht der Membran, die im allgemeinen mit Trägerschicht bezeichnet wird, kann grundsätzlich aus einem beliebigen, vorteilhaft ausgewählten anorganischen oder organischen Werkstoff bestehen. Es hat sich jedoch als vorteilhaft herausgestellt, auch die zweite Schicht aus einem Werkstoff auf der Basis eines Polymers auszuwählen.

Bei einer vorzugsweisen Ausgestaltung der zweiten Schicht der Membran besteht das Polymer aus Polyetherimid.

Es hat sich gezeigt, daß die Betriebssicherheit der erfindungsgemäßen Membran noch dadurch erhöht werden kann, daß vorzugsweise auf der ersten Schicht eine dritte hydrophobe Schicht angeordnet wird, wobei auch gemäß einer anderen Ausgestaltung der Membran die dritte Schicht auf der Permeatseite und/oder der Retentatseite der Membran angeordnet sein kann. Auch ist es möglich, die hydrophobe dritte Schicht beidseitig auf der ersten Schicht anzuordnen.

Die erfindungsgemäße Membran kann ebenso wie andere bekannte Membranen grundsätzlich als vorzugsweise Anwendung findende Flachmembran ausgebildet sein, sie kann jedoch auch für spezielle Anwendungen vorteilhafterweise als Hohlfaden-/Röhrenmembran ausgebildet sein, wobei die erste Schicht bei der letzteren Ausgestaltung der Membran, je nach Zielsetzung, entweder innen oder außen in Röhrchen angeordnet sein kann.

Das Massenverhältnis Metallsalz zu Polymer zur Ausbildung der ersten Schicht kann je nach gewünschter Selektivität der Membran und in Abhängigkeit von den gewünschten zu trennenden Stoffen in weiten Bereichen, vorzugsweise jedoch im Bereich von 0,1 bis 9 gewählt werden. Gute Ergebnisse in bezug auf die Selektivität haben Massenverhältnisse von vorzugsweise 1 bis 5 ergeben.

Zur Lösung der Aufgabe ist das erfindungsgemäße Verfahren dadurch gekennzeichnet, daß
a. zunächst die wasserhaltige Lösung des organischen Polymers mit der wäßrigen Metallsalzlösung gemischt wird,
b. daß nachfolgend die entstandene Mischung auf die zweite bestehende mikroporöse Schicht als erste Schicht aufgebracht wird und
c. anschließend in einem Trocknungsvorgang der wäßrige Anteil der Mischung nach Verfahrensschritt a. entfernt wird.

Die erste Schicht, gebildet aus dem Polymer und dem eingelagerten Metallsalz, kann dabei vorteilhafterweise durch Aufsprühen dieses Gemisches in der wäßrigen Phase auf die mikroporöse zweite Schicht aufgebracht werden. Es ist aber auch möglich, die mikroporöse zweite Schicht in die wäßrige Phase der ersten Schicht einzutauchen. Unabhängig von der Art des Aufbringens der ersten auf die zweite Schicht wird nach dem Verdunsten des Wassers auf der Oberfläche der mikroporösen zweiten Schicht eine dünne porenfreie erste Schicht des modifizierten Polyethylens erhalten.

Bei einer weiteren Ausgestaltung des Verfahrens wird vorteilhafterweise auf die Membran aus erster und zweiter Schicht auf der Permeatseite und/oder Retentatseite eine hydrophobe dichte Schicht aufgetragen, die bei einer weiteren Modifizierung des Verfahrens auch derart aufgetragen werden kann, daß beide Seiten der ersten Schicht eine hydrophobe dritte Schicht tragen.

Bei der letztgenannten Verfahrensmodifikation wird die hydrophobe dritte Schicht zunächst durch Tauchen oder Sprühen auf die zweite Schicht aufgetragen, dann wird auf die hydrophobe dritte Schicht die erste Schicht durch Sprühen oder Tauchen aufgetragen, wobei nachfolgend dann auf die noch freie Seite der ersten Schicht wiederum eine hydrophobe dritte Schicht durch Sprühen oder Tauchen aufgetragen wird.

Besondere Aspekte der Erfindung werden anhand der nachfolgenden schematischen Zeichnung beschrieben. Darin zeigen:
- Fig. 1: einen Buten/Butan-Fluß in Abhängigkeit vom Silbergehalt einer AgBF₄-Polyethylenmembran,
- Fig. 2: einen Buten/Butan-Fluß in Abhängigkeit vom Silbergehalt einer AgBF₄-Nafionmembran,
- Fig. 3: die Selektivität in Abhängigkeit vom Silbergehalt einer AgBF₄-Nafionmembran und
- Fig. 4: den Butenfluß in Abhängigkeit vom Permeatdruck durch eine AgBF₄-Nafionmembran.

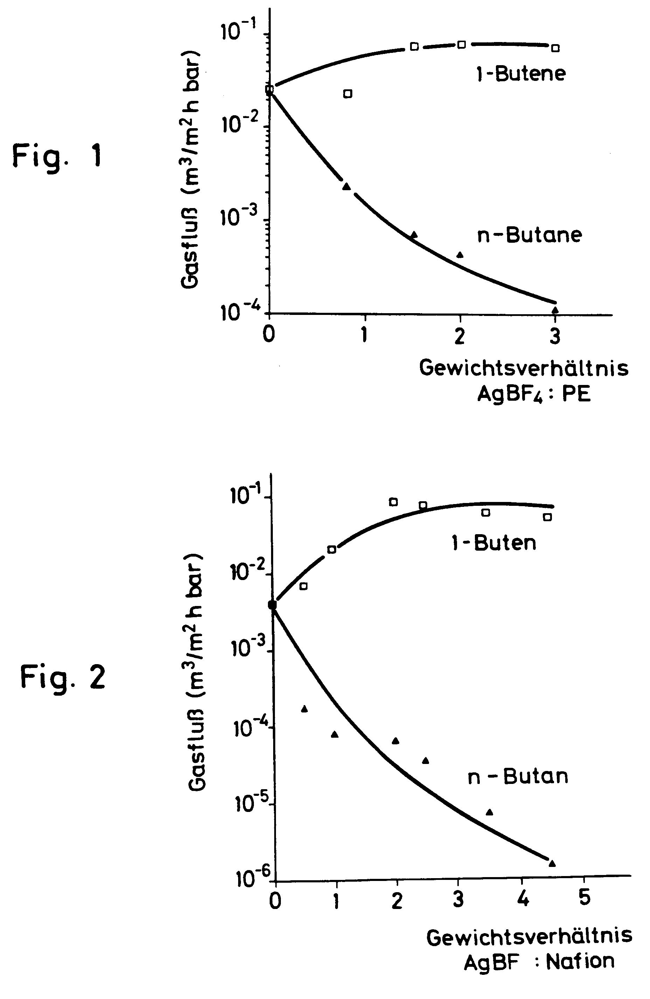

Fig. 1 zeigt die Flüsse von Butan und Buten durch eine erfindungsgemäße Membran, deren selektive Schicht aus (modifiziertem) Polyethylen als Polymer mit unterschiedlichem Salzgehalt besteht. Die Versuchsbedingungen waren: Feeddruck 1000 mbar, Permeatdruck < 10 mbar, Temperatur 35°C. Aus Fig. 1 ist ersichtlich, daß der Butenfluß um etwa den Faktor 3 ansteigt, wenn das Massenverhältnis AgBF₄ zu Polyethylen von 0 auf 3 erhöht wurde. Gleichzeitig wurde festgestellt, daß der Butananfluß durch die Membran um mehr als 2 Zehnerpotenzen fiel. Die resultierende Buten/Butan-Selektivität ist, wie erfindungsgemäß angestrebt, extrem hoch, und wird von keiner bisher bekannten Membran auch nur annähernd erreicht.

Wie erfindungsgemäß angestrebt, kann die Membran in wasserfreiem Zustand ohne Befeuchtung des Feedstromes eingesetzt werden. Wie schon erwähnt, können anstelle des Silbertetrafluoroborats viele andere Metallsalze in der Polyethylendispersion gelöst werden, die mit Olefinen reversibel reagieren. So kann beispielsweise das BF₄⁻ -Anion ersetzt werden durch ein Nitrat, ein Perchlorat oder ein Trifluormethansulfonat. Das Silber kann grundsätzlich durch andere Metalle ersetzt werden, wobei als die Metalle der ersten Reihe der Übergangselemente mit Ordnungszahlen von 21 bis 29 wie Chrom, Kupfer, insbesondere Kupfer(I)-ionen und Metalle der Eisengruppe wie Nickel und Eisen verwendet werden können. Weitere geeignete komplexbildende Metalle der zweiten und dritten Gruppe der Übergangselemente, d.h. Metalle mit Ordnungszahlen von 39 bis 47 oder 57 bis 79 sowie Quecksilber, insbesondere in Form der Quecksilber(I)-ionen, können zur Bildung der Metallsalze eingesetzt werden. Auch Edelmetalle wie Gold und die Elemente der Platingruppe wie Platin, Paladium, Rhodium, Ruthenium und Osmium sind geeignet. Auch verschiedene Kombinationen von Metallsalzen können verwendet werden.

Aus Fig. 2 sind die Flüsse von Butan und Buten durch die erfindungsgemäße Membran ersichtlich, deren selektive Schicht, d.h. die Schicht 1 aus Nafion mit unterschiedlichem Silbersalzgehalt besteht. Es ist ersichtlich, daß der Butanfluß um ungefähr den Faktor 13 ansteigt, wenn das Massenverhältnis AgBF₄ zu Nafion von 0 auf 4,5 erhöht wird. Gleichzeitig fällt der Butananfluß um den Faktor 2,7 x 10³ ab.

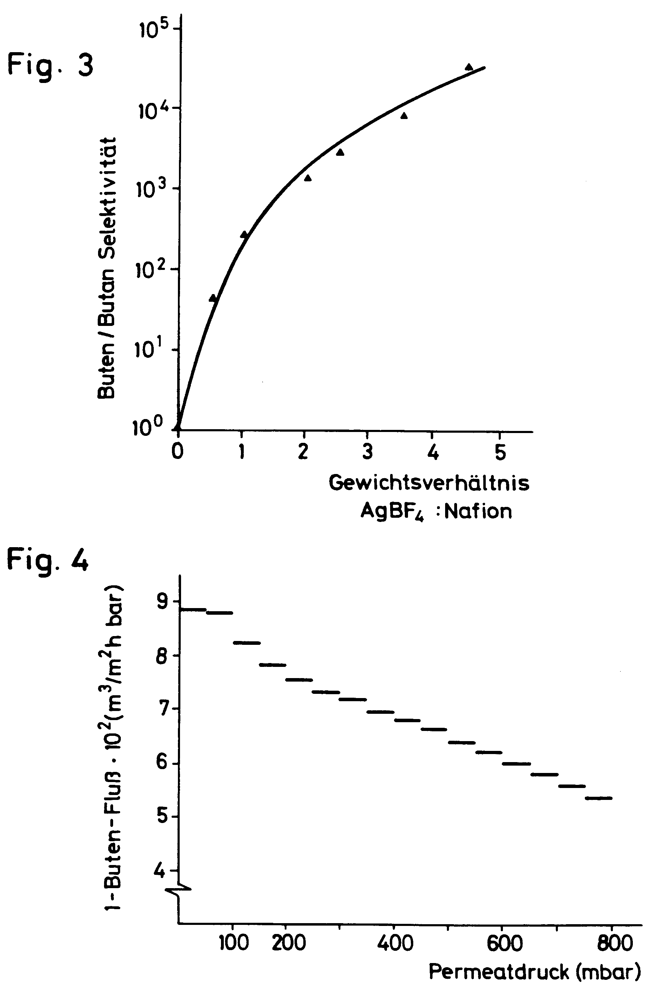

Fig. 3 zeigt die resultierende hohe Membranselektivität, die sich durch Division der beiden Flüsse auf Butan und Buten ergibt. Auch im Falle der nafionhaltigen Membran ist das Silbertetrafluoroborat nur beispielhaft anzusehen, d.h. es können im Prinzip alle geeigneten Metallsalze, wie erwähnt, verwendet werden.

Die meisten erleichterten Transport ermöglichenden Membranen funktionieren häufig nur dann, wenn der Partialdruck des abzutrennenden Gases auf der Permeatseite sehr niedrig gehalten wird. Dies kann entweder durch ein hohes Spülgasvolumen auf der Permeatseite geschehen oder indem ein hohes Vakuum permeatseitig erzeugt wird. Die erfindungsgemäß hergestellten Membranen benötigen hingegen keine starke Partialdruckabsenkung des abzutrennenden Olefins auf der Permeatseite.

Aus Fig. 4 ist schließlich der Butenfluß durch eine erfindungsgemäße Membran ersichtlich, deren selektive erste Schicht ca. 1,5 x 10⁻³ mm dick ist und aus AgBF ₄/Nafion besteht, aufgetragen gegen den Permeatdruck. Zwar fällt der Butenfluß mit steigendem Permeatdruck, aber auch bei verhältnismäßig hohen Permeatdrücken weist die erfindungsgemäße Membran noch einen sehr guten Fluß auf.

Die folgenden Beispiele dienen der weiteren Erläuterung der Erfindung.

### Beispiel 1

Man löst 5 g Silbertetrafluoroborat in einer Mischung aus 10 g Wasser, 1 g Wasserstoffperoxid und 0,5 g Glycerin. Diese Lösung mischt man mit 27,7 g Polyethylendispersion (Tegoglas Coating Agent, RP 30, Th. Goldschmidt AG, Essen). Mit dieser Mischung wird eine mikroporöse Membran durch kurzes Eintauchen beschichtet. Die mikroporöse Membran kann aus Polysulfon, Polyetherimid, Polyvinylidenfluorid oder anderen Polymeren bestehen. Die maximale Porengröße sollte 10⁻⁴ mm betragen, die minimale Luftpermeabilität 10 m³/m² h bar. Die Membran wird zunächst luftgetrocknet, dann für einige Stunden bei 70° C im Trockenschrank. Die entstandene selektive porenfreie Trennschicht ist etwa 3 x 10⁻³ mm dick und besteht zu 55,5 Gew.% aus modifiziertem Polyethylen und zu 44,5 Gew.% aus AgBF₄. Die Membran wird mit trockenem Butan unter einem Druck von 1013 mbar bei 35° C beschickt. Permeatseitig wird ein Vakuum von 10 mbar angelegt. Es resultiert ein Butanfluß von 2,3 x 10⁻³ m³/m² h bar. Der Butenfluß unter gleichen Bedingungen beträgt 2,3 x 10⁻² m³/m² h bar. Es ergibt sich eine ideale Selektivität von 10.

### Beispiel 2

Man stellt eine Membran entsprechend Beispiel 1 her mit dem Unterschied, daß anstelle von 5 g AgBF₄ 12,5 g gelöst werden. Die resultierende selektive Trennschicht besteht aus 66,7 Gew.% AgBF₄ und zu 33,3 Gew.% modifiziertem Polyethylen. Der Butanfluß beträgt unter den gleichen Bedingungen wie in Beispiel 1 4,3 x 10⁻⁴ m³/m² h bar, der Butenfluß beträgt 8,1 x 10⁻² m³/m² h bar. Es ergibt sich eine ideale Selektivität von 188.

### Beispiel 3

Die Membran aus Beispiel 2 wird bei 20° C mit einem trokkenen Gemisch aus Butan (93,6 %) und Buten (6,4%) beschickt. Der Feeddruck beträgt 1013 mbar und der Permeatdruck 16 mbar. Bei einem Stufenschnitt (Permeatfluß/ Feedfluß) kleiner 1 % erhält man eine Butenkonzentration im Permeat von 77,2 %. Das entspricht einer Buten/Butanselektivität der Membran von 62.

### Beispiel 4

Man stellt eine Membran entsprechend Beispiel 1 her mit dem Unterschied, daß anstelle von 5 g AgBF₄ 18,8 g AgBF₄ eingesetzt werden. Die resultierende selektive Schicht besteht jetzt aus 75 Gew.% AgBF₄ und 25 Gew.% modifiziertem Polyethylen. Der Butanfluß beträgt unter den gleichen Bedingungen wie in Beispiel 1 1,1 x 10⁻⁴ m³/m² h bar, der Butenfluß beträgt 7,6 x 10⁻² m³/m² h bar. Es errechnet sich eine ideale Selektivität von 691.

### Beispiel 5

6 g AgBF₄ werden in 24 g Wasser, 1,8 g Wasserstoffperoxid und 0,7 g Glycerin gelöst. Diese Lösung wird mit 240 g 5 %iger Nafionlösung (Solution Technology Inc. Mendenhall, PA 19357, USA) gemischt. Wie in Beispiel 1 wird eine mikroporöse Membran mit dieser Mischung beschichtet. Nach Verdampfen der Lösemittel entsteht eine porenfreie selektive Trennschicht, die ca. 2 x 10⁻³ mm dick ist und zu 33,3 % aus AgBF₄ und zu 66,7 % aus Nafion besteht. Die Membran wird mit trockenem Butan unter einem Druck von 1013 mbar bei 35° C beschickt. Permeatseitig wird ein Vakuum von 10 mbar erzeugt. Es resultiert ein Butanfluß von 1,67 x 10⁻⁴ m³/m² h bar. Der Butenfluß unter den gleichen Bedingungen beträgt 7,03 x 10⁻³ m³/m² h bar. Es resultiert eine ideale Buten/ Butan-Selektivität von 42.

### Beispiel 6

Man stellt eine Membran entsprechend Beispiel 5 her mit dem Unterschied, daß anstelle von 240 g Nafionlösung 48 g Nafionlösung eingesetzt werden. Die resultierende selektive Trennschicht besteht jetzt aus 71,4 Gew.% AgBF₄ und 28,6 Gew.% Nafion. Der Butanfluß beträgt unter den gleichen Bedingungen wie in Beispiel 5 3,51 x 10⁻⁵ m³/m² h bar und der Butenfluß 9,05 x 10⁻² m³/m² h bar. Es errechnet sich eine ideale Selektivität von 2577.

### Beispiel 7

Die Membran aus Beispiel 6 wird bei 20° C mit einem trockenen Gemisch aus Butan (53,4 %) und Buten (46,6 %) beschickt. Der Feeddruck beträgt 1013 mbar und der Permeatdruck 16 mbar. Bei einem Stufenschnitt kleiner 1 % erhält man eine Butenkonzentration im Permeat von 98,6 %. Das entspricht einer Buten/Butan-Selektivität der Membran von 85.

### Beispiel 8

Man stellt eine Membran entsprechend Beispiel 5 her mit dem Unterschied, daß anstelle von 240 g Nafionlösung 26,7 g Nafionlösung eingesetzt werden. Die resultierende Trennschicht besteht jetzt aus 81,8 Gew.% AgBF₄ und 18,2 Gew.% Nafion. Der Butanfluß beträgt unter den gleichen Bedingungen wie in Beispiel 5 1,51 x 10⁻⁶ m³/m² h bar und der Butenfluß 0,054 m³/m² h bar. Es errechnet sich eine ideale Buten/Butan-Selektivität der Membran von c. 35. 000.

### Beispiel 9

Die Membran aus Beispiel 8 wird bei 20° C einem Ethenstrom von 2 bar ausgesetzt. Permeatseitig herrscht ein Druck von 1 bar. Es ergibt sich ein Ethenfluß von 0,228 m³/m² h bar. Der Ethanfluß unter gleichen Bedingungen beträgt 3,46 x 10⁻⁴ m³/m² h bar. Es resultiert eine ideale Ethen/ Ethan-Selektivität von 659.

### Beispiel 10

Man löst 2,97 g CuCl und 1,61 g NH₄Cl in einer Mischung aus 30 g Wasser, 9 g 37 % HCl, 0,3 g Glycerin. Diese Lösung mischt man mit 39 g Diofan (Diofan 193 D, BASF, eine Copolymerdispersion von Vinylidenchlorid uns Acrylsäuremethylester) . Gemäß Beispiel 1 wird mit dieser Mischung eine Polyetherimidkompositmembran hergestellt, für die ein Polymer: CuCl-Verhältnis gleich 2,5 gilt. Butan- und Butadienfluß, gemessen gemäß Beispiel 1, sind jeweils 4,3 x 10⁻⁶ m³/m² h bar und 1,5 x 10⁻⁵ m³/m² h bar. Es ergibt sich eine ideale Selektivität von 3,4.

## Patentansprüche

1. Membran zum Austrag ungesättigter Kohlenwasserstoffe aus gasförmigen und flüssigen Stoffgemischen, insbesondere Kohlenwasserstoffgemischen, mit einer als Trenn- oder Sperrschicht dienenden ersten Schicht, die aus einer wasserhaltigen Lösung eines organischen Polymers und eines Metallsalzes erhältlich ist, sowie einer zweiten mikroporösen Schicht, deren Poren und/oder wenigstens Porenöffnungen von der ersten Schicht abgedeckt sind, dadurch gekennzeichnet, daß das organische Polymer aus einem Ionenaustauscher besteht und das Massenverhältnis Metallsalz zu organischem Polymer 0,1 bis 9 beträgt.

2. Membran nach Anspruch 1, dadurch gekennzeichnet, daß die erste Schicht die zweite Schicht vollständig bedeckt.

3. Membran nach einem oder beiden der Ansprüche 1 oder 2, dadurch gekennzeichnet, daß die erste Schicht 5 x 10⁻⁴ bis 10⁻² mm dick ist.

4. Membran nach einem oder beiden der Ansprüche 1 bis 3, dadurch gekennzeichnet, daß zur Bildung der ersten Schicht eine wäßrige Polymerlösung oder eine wäßrige Polymerdispersion, die das Metallsalz in gelöster Form enthalten, dient.

5. Membran nach einem oder mehreren der Ansprüche 1 bis 4, dadurch gekennzeichnet, daß das Polymer aus einer Polyolefindispersion gebildet wird.

6. Membran nach Anspruch 5, dadurch gekennzeichnet, daß das Polymer Polyvinylidenchlorid enthält.

7. Membran nach einem oder mehreren der Ansprüche 1 bis 6, dadurch gekennzeichnet, daß die Polymerlösung eine Nafionlösung ist.

8. Membran nach einem oder mehreren der Ansprüche 1 bis 7, dadurch gekennzeichnet, daß als Metallionen die Ionen der Edelmetalle des Periodensystems der Elemente ausgewählt sind.

9. Membran nach Anspruch 8, dadurch gekennzeichnet, daß die Metallionen Silbermetallionen sind.

10. Membran nach einem oder mehreren der Ansprüche 1 bis 9, dadurch gekennzeichnet, daß als Metallionen die Ionen der Nichtedelmetalle des Periodensystems der Elemente ausgewählt sind.

11. Membran nach Anspruch 10, dadurch gekennzeichnet, daß die Metallionen Kupfer(I)-ionen sind.

12. Membran nach Anspruch 10, dadurch gekennzeichnet, daß die Metallionen Zinkionen sind.

13. Membran nach einen oder mehreren der Ansprüche 1 bis 12, dadurch gekennzeichnet, daß als Anion Bortetrafluorid (BF₄-) ausgewählt wird.

14. Membran nach einem oder mehreren der Ansprüche 1 bis 13, dadurch gekennzeichnet, daß die zweite Schicht aus einem anorganischen Werkstoff besteht.

15. Membran nach einem oder mehreren der Ansprüche 1 bis 13, dadurch gekennzeichnet, daß die zweite Schicht aus einem Werkstoff auf der Basis eines Polymers besteht.

16. Membran nach Anspruch 15, dadurch gekennzeichnet, daß das Polymer Polyetherimid ist.

17. Membran nach einem oder mehreren der Ansprüche 1 bis 16, dadurch gekennzeichnet, daß auf der ersten Schicht eine dritte hydrophobe Schicht angeordnet ist.

18. Membran nach einem oder mehreren der Ansprüche 1 bis 17, dadurch gekennzeichnet, daß die dritte Schicht auf der Permeatseite und/oder der Retentatseite der Membran angeordnet ist.

19. Membran nach einem oder beiden der Ansprüche 17 oder 18, dadurch gekennzeichnet, daß auf der ersten Schicht beidseitig die dritte Schicht angeordnet ist.

20. Membran nach einem oder mehreren der Ansprüche 1 bis 19, dadurch gekennzeichnet, daß diese als Flachmembran ausgebildet ist.

21. Membran nach einem oder mehreren der Ansprüche 1 bis 19, dadurch gekennzeichnet, daß diese als Hohlfaden-/ Röhrenmembran ausgebildet ist.

22. Membran nach einem oder mehreren der Ansprüche 1 bis 21, dadurch gekennzeichnet, daß in der ersten Schicht das Massenverhältnis Metallsalz zu Polymer 1 bis 5 beträgt.

23. Verfahren zur Herstellung einer Membran zum Austrag ungestättigter Kohlenwasserstoffe aus gasförmigen und flüssigen Stoffgemischen, insbesondere Kohlenwasserstoffgemischen, nach einem oder mehreren der Ansprüche 1 bis 22, dadurch gekennzeichnet, daß
a. zunächst die wasserhaltige Lösung des organischen Polymers mit der wäßrigen Metallsalzlösung gemischt wird,
b. nachfolgend die entstandene Mischung auf die zweite bestehende mikroporöse Schicht als erste Schicht aufgebracht wird und
c. anschließend in einem Trocknungsvorgang der wäßrige Anteil der Mischung nach Verfahrensschritt a. entfernt wird.

24. Verfahren nach Anspruch 23, dadurch gekennzeichnet, daß auf der Membran aus erster und zweiter Schicht auf der Permeatseite und/oder der Retentatseite eine hydrophobe dritte Schicht aufgetragen wird.

25. Verfahren nach einem oder beiden der Ansprüche 23 oder 24, dadurch gekennzeichnet, daß auf beide Seiten der ersten Schicht eine hydrophobe dritte Schicht aufgetragen wird.

## Claims

1. A membrane for extracting unsaturated hydrocarbons from gaseous and liquid mixtures of materials, particularly hydrocarbon mixtures, with a first layer serving as a separating or barrier layer, which can be obtained from an aqueous solution of an organic polymer and a metal salt, as well as a second microporous layer, whose pores and/or pore openings at least are covered by the first layer, characterized in that the organic polymer consists of an ion exchanger, and the mass ratio of metal salt to organic polymer amounts to 0.1 to 9.

2. A membrane according to Claim 1, characterized in that the first layer covers the second layer completely.

3. A membrane according to one or both Claims 1 or 2, characterized in that the first layer is 5 x 10⁻⁴ to 10⁻² mm thick.

4. A membrane according to one or more of Claims 1 to 3, characterized in that an aqueous polymer solution or an aqueous polymer dispersion containing the metal salt in dissolved form, serves for the formation of the first layer.

5. A membrane according to one or more of Claims 1 to 4, characterized in that the polymer is formed from a polyolefine dispersion.

6. A membrane according to Claim 5, characterized in that the polymer contains polyvinylidene chloride.

7. A membrane according to one or more of Claims 1 to 6, characterized in that the polymer solution is a Nafion solution.

8. A membrane according to one or more of Claims 1 to 7, characterized in that ions of noble metals in the Periodic system of elements are selected as metal ions.

9. A membrane according to Claim 8, characterized in that the metal ions are silver metal ions.

10. A membrane according to one or more of Claims 1 to 9, characterized in that the ions of non-noble metals in the Periodic system of elements, are selected as metal ions.

11. A membrane according to Claim 10, characterized in that the metal ions are copper (I)-ions.

12. A membrane according to Claim 10, characterized in that the metal ions are zinc ions.

13. A membrane according to one or more of Claims 1 to 12, characterized in that boron tetrafluoride (BF₄-) is selected as an anion.

14. A membrane according to one or more of Claims 1 to 13, characterized in that the second layer consists of an inorganic material.

15. A membrane according to one or more of Claims 1 to 13, characterized in that the second layer consists of a material on the basis of a polymer.

16. A membrane according to Claim 15, characterized in that the polymer is polyetherimide.

17. A membrane according to one or more of Claims 1 to 16, characterized in that a third hydrophobic layer is disposed on the first layer.

18. A membrane according to one or more of Claims 1 to 17, characterized in that the third layer is disposed on the permeate and/or retentate side of the membrane.

19. A membrane according to one or both Claims 17 and 18, characterized in that the third layer is disposed on both sides of the first layer.

20. A membrane according to one or more of Claims 1 to 19, characterized in that this is constructed as a flat membrane.

21. A membrane according to one or more of Claims 1 to 19, characterized in that this is constructed as a hollow thread-/tubular membrane.

22. A membrane according to one or more of Claims 1 to 21, characterized in that the mass ratio of metal salt to polymer amounts to 1 to 5 in the first layer.

23. A process for manufacturing a membrane for extracting unsaturated hydrocarbons from gaseous and liquid mixtures of materials, particularly hydrocarbon mixtures according to one or more of Claims 1 to 22, characterized in that
a. first of all the aqueous solution of the organic polymer is mixed with the aqueous metal salt solution,
b. following this, the resulting mixture is introduced on to the second existing microporous layer as a first layer and
c. subsequently, the aqueous part of the mixture is removed in a drying procedure after process step a.

24. A process according to Claim 23, characterized in that a third hydrophobic layer is applied on the permeate and/or retentate side of the membrane made of the first and second layers.

25. A process according to one or both Claims 23 or 24, characterized in that a third hydrophobic layer is applied on both sides of the first layer.

## Revendications

1. Membrane pour l'extraction d'hydrocarbures insaturés à partir de mélanges de matières gazeuses ou liquides, plus particulièrement des mélanges d'hydrocarbures, munie d'une première couche servant de couche de séparation ou d'arrêt, que l'on peut obtenir à partir d'une solution aqueuse d'un polymère organique et d'un sel métallique, ainsi que d'une deuxième couche microporeuse, dont les pores et/ou au moins les orifices de pores son recouvert par la première couche, caractérisée en ce que le polymère organique est constitué d'un échangeur d'ions et le rapport massique du sel métallique au polymère organique est de 0,1 à 9.

2. Membrane selon la revendication 1, caractérisée en ce que la première couche couvre complètement la deuxième couche.

3. Membrane selon l'une ou les deux revendications 1 ou 2, caractérisée en ce que la première couche a une épaisseur de 5x10⁻⁴ à 10⁻² mm.

4. Membrane selon l'une ou les deux revendications 1 ou 2, caractérisée en ce que pour la formation de la première couche, on se sert d'une solution aqueuse de polymère ou d'une dispersion aqueuse de polymère qui contient le sel métallique sous forme dissoute.

5. Membrane selon une ou plusieurs des revendications 1 à 4, caractérisée en ce que le polymère est formé à l'aide d'une dispersion aqueuse de polyoléfine.

6. Membrane selon la revendication 5, caractérisée en ce que le polymère contient le poly(chlorure de vinylidène).

7. Membrane selon une ou plusieurs des revendications 1 à 6, caractérisée en ce que la solution de polymère est une solution de Nafion.

8. Membrane selon une ou plusieurs des revendications 1 à 7, caractérisée en ce que les ions métalliques sont pris parmi les ions métaux nobles du système de classification périodique des éléments.

9. Membrane selon la revendication 8, caractérisée en ce que les ions métalliques sont des ions argent métal.

10. Membrane selon une ou plusieurs des revendications 1 à 9, caractérisée en ce que les ions métalliques sont pris parmi les ions métaux ordinaires du système de classification périodique des éléments.

11. Membrane selon la revendication 10, caractérisée en ce que les ions métalliques sont les ions cuivre(I).

12. Membrane selon la revendication 10, caractérisée en ce que ions métalliques sont les ions zinc.

13. Membrane selon une ou plusieurs des revendications 1 à 12, caractérisée en ce qu'on prend comme anion le tétrafluorure de bore (BF₄⁻).

14. Membrane selon une ou plusieurs des revendications 1 à 13, caractérisée en ce que la deuxième couche est constitué d'un matériau inorganique.

15. Membrane selon une ou plusieurs des revendications 1 à 13, caractérisée en ce que la deuxième couche est constituée d'un matériau à base d'un polymère.

16. Membrane selon la revendication 15, caractérisée en ce que le polymère est le polyétherimide.

17. Membrane selon une ou plusieurs des revendications 1 à 16, caractérisée en ce que sur la première couche est disposée une troisième couche hydrophobe.

18. Membrane selon une ou plusieurs des revendications 1 à 17, caractérisée en ce que en ce que la troisième couche est disposée sur la face perméat et/ou retentat de la membrane.

19. Membrane selon une ou les deux revendications 17 ou 18, caractérisée en ce qu'une troisième couche est disposée sur les deux faces de la première couche.

20. Membrane selon une ou plusieurs des revendications 1 à 19, caractérisée en ce qu'elle est formée comme membrane plate.

21. Membrane selon une ou plusieurs des revendications 1 à 19, caractérisée en ce qu'elle est constituée comme membrane de fibres creux ou membrane tubulaire.

22. Membrane selon une ou plusieurs des revendications 1 à 21, caractérisée en ce que dans la première couche le rapport massique du sel métallique au polymère est de 1 à 5.

23. Procédé pour la préparation d'une membrane pour l'extraction d'hydrocarbures insaturés à partir de mélanges de matières gazeuses ou liquides, notamment des mélange d'hydrocarbures selon une ou plusieurs des revendications 1 à 22, caractérisé en ce que
a) on mélange d'abord la solution aqueuse du polymère organique avec la solution aqueuse des sels métalliques,
b) par la suite, on dépose le mélange formé, comme première couche, sur la deuxième couche microporeuse existante et
c) ensuite, on élimine à l'aide d'un procédé par voie sèche la portion aqueuse du mélange selon l'étape a).

24. Procédé selon la revendication 23, caractérisé en ce qu'on dépose sur la membrane constituée de la première et de la deuxième couche, sur la face perméat et/ou retentat, une troisième couche hydrophobe.

25. Procédé selon une ou les deux revendications 23 ou 24, caractérisé en ce qu'on dépose une troisième couche hydrophobe sur les deux faces de la première couche.
